# EUROPEAN PATENT APPLICATION

(11) **EP 4 631 548 A2**
(43) Date of publication of application: **15.10.2025**
(21) Application number: 25198574.3
(22) Date of filing: 29.05.2020
(51) Int. Cl.: A61M 5/14

(54) **RADIATION OR ANTICANCER CHEMOTHERAPY SENSITIZER ADMINISTRATION HOLDER**

(30) Priority: 31.05.2019 JP 2019102703
(62) Divisional of application: 20814397.4
(71) Applicant: Kortuc Japan LLC, Tokyo 1056004 (JP); Taisei Kako Co., Ltd., Kita-ku Osaka-shi Osaka 531-0072 (JP)
(72) Inventor: Yamashita, Shogo, Tokyo, 105-6004 (JP); Horita, Taiji, Osaka, 567-0054 (JP); Sonoyama, Tomoyuki, Osaka, 567-0054 (JP)
(74) Representative: Gulde & Partner

(57) **Abstract**

Mixing a low-viscosity solution and a high-viscosity solution was difficult. An object of the present invention is to provide a holder for mixed administration of at least two types of solutions for enhancing the radiation or anticancer therapeutic effect on a tumor, wherein the holder is capable of simultaneously discharging each solution at a constant ratio from respective syringes containing each solution.

## Description

### Technical field

The present invention is a holder for mixing and administrating at least two solutions, in particular a holder capable of simultaneously ejecting each solution in a fixed ratio from each syringe containing each solution.

### Background

Surgery is the most common local treatment method for malignant tumors, and radiotherapy is the second most common treatment method. The number of patients treated with the radiotherapy has increased dramatically in recent years due to its applicability to elderly patients and its ability to preserve normal organs and tissues. However, high-energy X-rays and electron beams from linear accelerators, which are commonly used in these radiotherapies, are low linear energy transfer (LET) radiation and have relatively lower biological effectiveness. Therefore, the linear accelerator radiation therapy for tumors such as malignant melanoma, various types of sarcomas, and pleomorphic glioblastoma is ineffective. Locally advanced cancers that have grown to more than a few centimeters in size are less effective for the linear accelerator radiation therapy due to the high number of hypoxic tumor cells and the large amount of antioxidant enzymes they contain, which make them radioresistant.

Patent document 1 discloses a technique in which hydrogen peroxide is combined with hyaluronic acid or a salt thereof to effectively exert a radiation sensitization effect and an anticancer chemotherapy sensitization effect. Patent document 2 discloses a technique in which hydrogen peroxide is combined with a hydrogel containing a cross-linked gelatin gel prepared from acidic gelatin to effectively exert a radiation sensitization effect and an anticancer chemotherapy sensitization effect.

### Prior arts

### Patent Documents

Patent Document 1: WO2008/041514
Patent Document 2: WO2014/126222

### Summary of the Invention

### Problem to be Solved by the Invention

However, the technologies disclosed by Patent documents 1 and 2 take a very long time to mix hyaluronic acid or hydrogel with hydrogen peroxide solution due to their high viscosity. In addition, when the hyaluronic acid or hydrogel is mixed with the hydrogen peroxide solution in advance, the hydrogen peroxide is decomposed.

### Means for Solving the Problem

The inventors have developed a technique that makes it possible to easily mix low and high viscosity solutions at the time of use and to easily change the ratio of low and high viscosity solutions in the mixture, and then the invention has been completed.

It is an object of the present invention to provide a holder for mixing and administering at least two solutions to enhance the effectiveness of treatment of tumors with radiation or anticancer agents, wherein the holder is capable of simultaneously ejecting each solution in a fixed ratio from each syringe containing each solution.

Such a holder allows plunger rods of each syringe to be pushed simultaneously, so that each solution can be simultaneously ejected in a fixed ratio from each syringe containing each solution.

The present invention also provides the holder including a housing section for housing the syringes, a connecting section for connecting the syringes to the holder, an ejection section for ejecting the solution, and a flow channel in fluid communication with the ejection section and the connecting section.

Another object of the present invention is to provide a drug delivery device, including syringes and the holder.

The present invention also provides the drug delivery device in which cross-sectional areas of filling spaces in each syringe differ from each other.

The present invention also provides the drug delivery device, further including an adapter, in which each syringe is equipped with a plunger rod and, the adapter is connected to the plunger rods.

The present invention also provides the drug delivery device in which each syringe is pre-filled with a different desired solution from each other.

The present invention also provides the drug delivery device in which at least one of the syringes is prefilled with a hydrogen peroxide solution, and material of a barrel of the syringe containing the hydrogen peroxide solution is a cyclo-olefin polymer (COP) or a cyclo-olefin copolymer (COC).

Another object of the present invention is to provide a kit including syringes and the holder.

The present invention also provides the kit in which each syringe is prefilled with a different desired solution from each other.

The present invention also provides the kit in which at least one of the syringes is prefilled with a hydrogen peroxide solution, and material of a barrel of the syringe containing the hydrogen peroxide solution is a cyclo-olefin polymer (COP) or a cyclo-olefin copolymer (COC).

### Brief Description of the Drawing

FIG. 1 illustrates a drug delivery device 1 according to the present embodiment.
FIG. 2 shows a graph of the residual rate of hydrogen peroxide remaining in each syringe due to the difference in the material of each syringe barrel in the example.

### Description of Embodiments

### Definition.

For convenience, certain terms employed in the context of the present disclosure are collected here. Unless defined otherwise, all technical and scientific terms used herein have the same meaning as commonly understood by one of the ordinary skilled in the art to which this invention belongs. The singular forms "a", "and", and "the" are used herein to include plural referents unless the context clearly dictates otherwise.

Notwithstanding that the numerical ranges and parameters setting forth the broad scope of the invention are approximations, the numerical values set forth in the specific examples are described as precisely as possible. Any numerical value, however, inherently contains certain errors necessarily resulting from the standard deviation found in the respective testing measurements. Also, as used herein, the term "about" generally means within 10 %, 5%, 1%, or 0.5 % of a given value or range. Alternatively, the term "about" means within an acceptable standard error of the mean when considered by one of ordinary skill in the art.

In order to avoid redundancy, explanation for similar contents is not repeated.

As an embodiment, there is provided a holder for mixing and administering at least two solutions to enhance the effectiveness of treatment of tumors with radiation or anticancer agents, in which the holder is capable of simultaneously ejecting each solution in a fixed ratio from each syringe containing each solution.

As another embodiment, there is provided a drug delivery device including syringes and the holder.

### Drug Delivery System

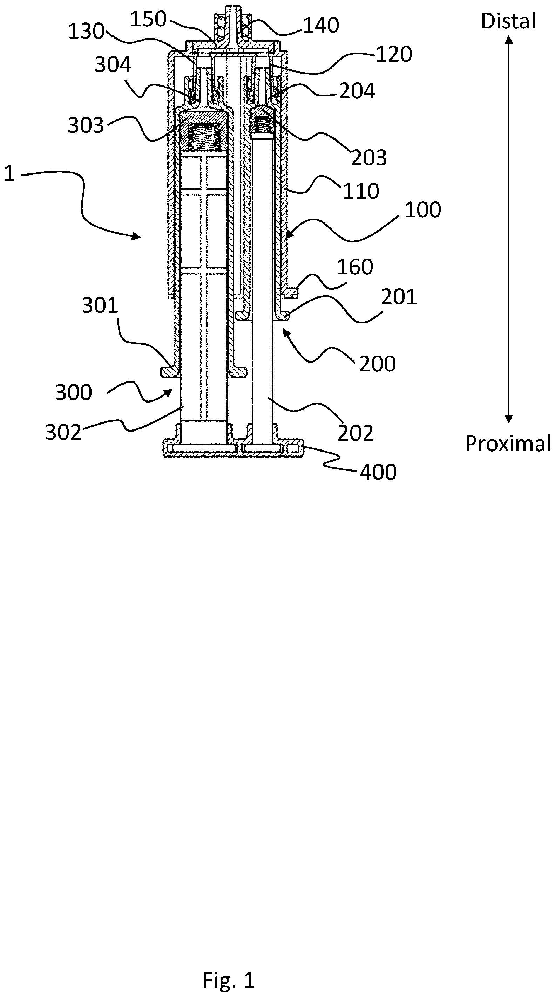

FIG. 1 shows a drug delivery device 1. The drug delivery device 1 includes a holder 100, syringes 200 and 300 housed in and connected to the holder 100, and an adapter 400 connected to a plunger rod 202 of the syringe 200 and a plunger rod 302 of the syringe 300. The drug delivery device 1 is manually operated but can also be used in conjunction with an appropriate electrical dosing device or mechanical dosing device. In the present specification, the holder 100 side of the drug delivery device 1 may be described as "distal" and the adapter 400 side of the drug delivery device 1 may be described as "proximal". For example, it may be described as "the distal end of the plunger rods 202 and 302 are provided with gaskets 203 and 303, respectively". The drug delivery device may be the one for mixing at least two solutions (e.g., a hydrogen peroxide solution and hyaluronic acid).

### Holder

The holder 100 has a housing section 110 for housing the syringes, a connecting sections 120 and 130 for connecting syringes 200 and 300 to the holder 100, an ejection section 140 for ejecting the solution, and a flow channel 150 in fluid communication with the connecting sections 120 and 130 and the ejection section 140. The connecting section 120 is in fluid communication with the flow channel 150. The connecting section 130 is in fluid communication with the flow channel 150. The ejection section 140 is in fluid communication with the flow channel 150. Therefore, the connecting section 120, the connecting section 130 and the ejection section 140 are in fluid communication with each other via the flow channel 150. The flow channel 150 may be a T-shaped flow channel or a Y-shaped flow channel. When the flow channel 150 is the T-shaped channel, flow direction of the solution from the connecting section 120 is opposed to flow direction of the solution from the connecting section 130, resulting in a more uniform mixing of the solutions. When the flow channel 150 is the Y-shaped flow channel, the confluence of the solution from the connecting section 120 and the solution from the connecting section 130 is smooth, resulting in smooth ejection of the mixed solution.

The housing section 110 of the holder 100 is capable of housing a plurality of syringes (syringe groups). In the housing section 110, each syringe is preferably configured to be arranged parallel to each other. The housing section 110 can hold or fix the syringes. The housing section 110 may be configured to hold and/or fix a group of syringes that all have the same outer diameter, or it may be configured to hold or fix a group of syringes in which the outer diameter of at least one of the plurality of syringes is different from the outer diameter of the other syringes (i.e., a group of syringes including a group of syringes having various outer diameters). The housing section 110 of the holder 100 has a plurality of sub-housing sections, and each sub-housing section may hold a single syringe or a plurality of syringes. Each sub-housing section may be configured to hold or fix syringes that have different outer diameters from each other. The housing section 110 does not need to cover the entire barrel of the syringe and may be configured to cover part of the barrels of the syringes.

Materials of the holder 100 may include, but are not limited to, metals (e.g., stainless steel), resins (e.g., cyclo-olefin polymers (COPs), cyclo-olefin copolymers (COCs), polypropylene and polycarbonate), and glass. The holder 100 may be manufactured from a single material or from a plurality of materials. The holder 100 may include a plurality of parts (composed of the same or different materials) or may be made from a single part. The flow channel 150 may be a tunnel inside the holder 100, or it may be a flexible tube (e.g., a silicone tube). The ejection section 140 may be fitted with a needle when in use, but an extension component (e.g., a silicone tube) may also be fitted between the ejection section 140 and the needle.

The holder 100 may have an opening (window) for checking the connecting sections 120 and 130. The proximal end of the holder 100 may be provided with a flange 160 for stable injection.

### Syringe

The syringes 200 and 300 include barrels 201 and 301 and plunger rods 202 and 302 with gaskets 203 and 303, respectively, wherein the barrels 201 and 301 have tips 204 and 304 that eject the solutions in the syringes 200 and 300, respectively. In FIG. 1, the syringes 200 and 300 are housed in the housing section 110 of the holder 100. The syringe 200 is detachably connected to the connecting section 120 of the holder 100 via the tip 204 of the syringe 200. The syringe 300 is detachably connected to the connecting section 130 of the holder 100 via the tip 304 of the syringe 300. The syringes 200 and 300 may be cartridge-type syringes in which the plunger rods and gaskets are non-fixed. Each syringe may also include a different desired solution from each other. The solutions may be pre-filled in each syringe and may be filled into each syringe when needed. The cross-sectional areas of the filling space in each syringe may be the same or different from each other. The cross-sectional area of the filling space in at least one of the plurality of syringes may be different from the cross-sectional area of the filling space of the other syringes. The expression "the cross-sectional area of the filling space in each syringe" means the cross-sectional area of the filling space with respect to an axis extending in the sliding direction of the plunger rod. When the cross-sectional area of the filling space in the syringe is disc shaped, the inner diameter of the syringe may be used instead of the cross-sectional area of the filling space in the syringe. The "filling space of the syringe" means the space of the syringe in which the solution is filled.

The syringes 200 and 300 may be manufactured from a single material or may be configured by multiple materials (including a multi-layered structure such as a coating). For a syringe manufactured from a single material, the entire syringe may be made of a resin (e.g., cyclo-olefin polymer (COP), cyclo-olefin copolymer (COC) and polypropylene). As discussed in the following example, a syringe that contains or may contain a hydrogen peroxide solution is preferably made of cyclo-olefin polymer (COP), cyclo-olefin copolymer (COC), or polypropylene. In the case of a syringe composed of more than one material, a portion of the syringe in direct contact with the solution may be made of the above-mentioned resin, while the other portion may be made of glass or metal. It is not necessary that all parts in contact with the solution are made of the above resin, but only if the main part of the syringe body, including the inner surface of the barrel of the syringe body, is made of the above resin. The plunger rod, the luer lock, the cap, the gasket and the like need not be made of the above resin.

### Adapter

The adapter 400 may be detachably connected to the plunger rods 202 and 302 and may be molded as one piece with the plunger rods 202 and 302. The adapter 400 may be connected to the proximal end of the plunger rods 202 and 302, or may be connected to the distal side of the plunger rods 202 and 302 rather than the proximal end of the plunger rods 202 and 302. When these are close enough to each other that the plunger rod 202 and the plunger rod 302 can be pushed at the same time, the adapter 400 may not be used. The adapter 400 may be connected to the holder 100 via a sliding mechanism (e.g., a rail) or a wire (e.g., a metal wire and a plastic wire).

### Solution(s)

In this embodiment, one solution is mixed with one or more other solutions upon use. If necessary, the mixed solution may be injected (e.g., by using the adapter 400 attached to the proximal end of the plunger rods 202 and 302) after one of the multiple solutions has been injected first. One of the two solutions may be a hydrogen peroxide solution and the other solution may be selected from the group consisting of hyaluronic acid, hydrogel and combinations thereof. In another embodiment, at least one of the above syringes is pre-filled with a hydrogen peroxide solution. When the hydrogen peroxide solution is pre-filled in the syringe, the material of the barrel of the syringe for the hydrogen peroxide solution is preferably a cyclo-olefin polymer (COP) or a cyclo-olefin copolymer (COC). When more than three solutions are used, a solution other than the above (e.g., Hanks' solution, Ringer's solution, saline, acetate buffer (to reduce discomfort at the injection site), suspension, stabilizer and dispersant) can be used.

The hydrogen peroxide solution means a solution of hydrogen peroxide dissolved in a solvent (e.g., water) and may contain additives (e.g., phosphoric acid and phenacetin) as needed. Concentration of hydrogen peroxide in the hydrogen peroxide solution is from 0.01 to 40% (w/v), for example, may be 0.01, 0.02, 0.03, 0.04, 0.05, 0.06, 0.07, 0.08, 0.09, 0.1, 0.5, 1, 5, 10, 15, 15, 20, 25, 25, 30, 30, 35 or 40%, or a value within a range between any two of the above values, preferably from 0.05 to 30% (w/v).

Hyaluronic acid includes salts thereof. The characteristics of hyaluronic acid (molecular weight, degree of crosslinking, etc.) used in the treatment of tumors by radiation or anticancer agent may be those known in the art (see, for example, WO2008/041514). The hydrogel containing the gelatin gel can be composed of, as the main component, a gelatin gel which is produced by cross-linking the gelatin by a chemical cross-linking agent, heat treatment or UV irradiation or electron beam irradiation. The gelatin gel may be an acid gelatin gel. The characteristics of the hydrogel containing the gelatin gel used in the treatment of tumors by radiation or anticancer (such as the type of gelatin, isoelectric point, content, degree of cross-linking, etc.) may be those known in the art (see, for example, WO2014/126222).

### Ratio of Solutions

The respective solutions in each syringe 200 or 300 flow into the flow channel 150 by pushing the adapter 400 toward the holder 100. The solutions are mixed in the flow channel 150. The mixed solutions are ejected from the ejection section 140. By using the adapter 400, the speed of movement of the plunger rod 202 of the syringe 200 is the same as that of the plunger rod 302 of the syringe 300, allowing each solution to be ejected in a fixed ratio. The proportion of each solution in the mixed solution is the proportion of the cross-sectional area in the filling space of each syringe. In other words, the proportion of each solution in the mixed solution depends on the ratio of the cross-sectional areas in the filling spaces of each syringe.

### Kit

As yet another embodiment, there is provided a kit including the above syringes and the above holder.

The kit may include a plurality of containers, each of which may contain the required amount of the desired solution. The kit may include an adapter 400. The kit may include one or more needles for the syringe and/or the holder. The kit may include additional elements (e.g., instructions or dosing plan) for treating tumors with radiation or anticancer agent. If the solution is pre-filled in the syringes 200 and 300, the tips 204 and 304 of the syringes are covered with caps.

### EXAMPLE

### Stability test of hydrogen peroxide solution

Stability test of a hydrogen peroxide solution was performed using a glass syringe, a COP syringe, and a COC syringe. 1 mL of the hydrogen peroxide solution was added to each syringe, sealed, and then stored at 60 °C for 4 weeks. The residual rates of hydrogen peroxide in the hydrogen peroxide solutions after storage were measured. Oxydol "KENEI" (containing 2.5 to 3.5%(w/v) hydrogen peroxide, phosphoric acid and phenacetin) manufactured by Kenei Pharmaceutical Co., Ltd. was used as the hydrogen peroxide solution. The amount of hydrogen peroxide in the hydrogen peroxide solution was detected by titration with a potassium permanganate solution according to oxydol determination method described in the Japanese Pharmacopoeia.

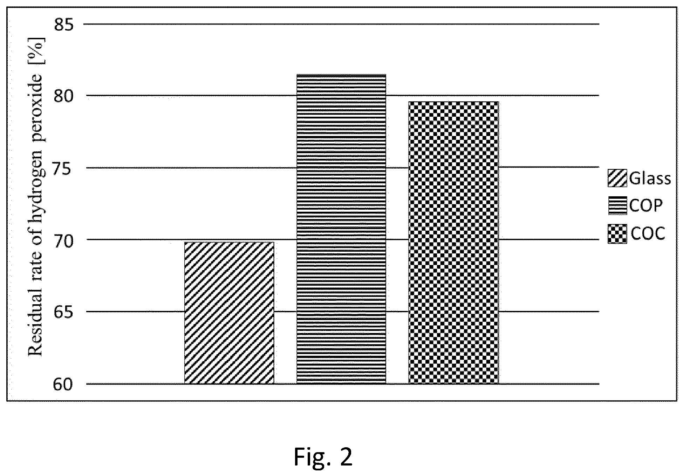

The results are shown in FIG. 2. In the case of the glass syringe, the residual rate of hydrogen peroxide was less than 70%, while the residual rate regarding COP syringe or COC syringe was 70% or more. As a result, the COP syringe and COC syringe were able to suppress the decomposition of hydrogen peroxide more than the glass syringe.

### EXPLANATION OF REFERENCES

- 1: Drug delivery system
- 100: Holder
- 110: Housing section
- 120, 130: Connecting section
- 140: Ejection section
- 150: Flow channel
- 160: Flange
- 200,300: Syringe
- 201,301: Barrel
- 202, 302: Plunger rod
- 203, 303: Gasket
- 204, 304: Tip.
- 400: Adapter

## Claims

1. A drug delivery device (1), comprising at least two syringes (200, 300) and a holder (100) for mixing and administering at least two solutions to enhance the effectiveness of treatment of tumors with radiation or anticancer agent,
wherein the holder (100) is capable of simultaneously ejecting solution containing each solution in a fixed ratio from each syringe (200, 300),
the holder (100) comprises a housing section (110) for housing the syringes (200, 300), connecting sections (120, 130) for connecting the syringes (200, 300) to the holder (100), an ejection section (140) for ejecting the solution, and a flow channel (150) in fluid communication with the ejection section (140) and the connecting sections (120, 130),
cross-sectional areas of filling spaces in each syringe (200, 300) differ from each other, and
**characterized in that**
at least one of the syringes (200, 300) is prefilled with a hydrogen peroxide solution.

2. The drug delivery device (1) according to claim 1, further comprising an adapter (400),
wherein each syringe (200, 300) is equipped with a plunger rod (202, 302) and,
the adapter (400) is connected to the plunger rods (202, 302).

3. The drug delivery device (1) according to claim 1 or 2, wherein each syringe (200, 300) is pre-filled with a different desired solution from each other.

4. A kit comprising the drug delivery device (1) according to claim 1.

5. The kit according to claim 4, wherein each syringe (200, 300) is prefilled with a different desired solution from each other.
